# EUROPEAN PATENT APPLICATION

(11) **EP 4 212 047 A1**
(43) Date of publication of application: **19.07.2023**
(21) Application number: 21893579.9
(22) Date of filing: 26.09.2021
(51) Int. Cl.: A24F 47/00, G01L 13/00

(54) **ELECTRONIC ATOMIZATION DEVICE HAVING DIGITAL AIR PRESSURE SENSING CHIP, AND CONTROL METHOD THEREFOR**

(30) Priority: 20.11.2020 CN 202011307299
(71) Applicant: Huizhou Happy Vaping Technology Limited, Huizhou, Guangdong 516000 (CN)
(72) Inventor: LIN, Guangrong, Guangdong 516000 (CN); ZHENG, Xianbin, Guangdong 516000 (CN); ZHANG, Xiyong, Guangdong 516000 (CN)
(74) Representative: Meyer, Thorsten
(86) International application number: PCT/CN2021/120590
(87) International publication number: WO 2022/105428

(57) **Abstract**

The disclosure provides an electronic vaporizing device with digital air pressure sensor chip and a controlling method thereof. The device comprises a vaporizer part arranged with a vaporizing unit, a power output unit, a microcontroller, and a digital air pressure sensor chip. The digital air pressure sensor chip is disposed in the air inlet channel of the vaporizer part, and comprises an air pressure sensing unit, an air pressure processing unit, a switching signal output unit, and a digital signal output unit. The air pressure sensing unit is configured to detect the air pressure difference. The switching signal output unit is configured to output a switch signal to activate the microcontroller which is in a sleep state, when the air pressure difference value reaches the set activation threshold. The output switch unit is configured to control the power output unit to provide or not provide output voltage when the air pressure difference value reaches or is lower than the set working threshold. It has advantages that, the digital air pressure sensor chip is set with the sleep frequency to save power consumption, with the activation threshold to avoid false triggering, and is arranged with the air pressure sensing unit to accurately detect the air pressure difference so as to achieve precise adjustment for vapor volume.

## Description

### TECHNICAL FIELD

The disclosure relates to the technical field of electronic vaporizing device, and more particularly, to an electronic vaporizing device with a digital air pressure sensor chip and a controlling method of the electronic vaporizing device with the digital air pressure sensor chip.

### BACKGROUND

Usually, an electronic vaporizing device includes a battery part and a vaporizer part. A battery cell inside the battery part is to supply power to the vaporizer part. The vaporizer part includes a vaporizing unit which can atomize the to-be-vaporized liquid or material into vapor or aerosol for users to vape when powered on. In particular, the electronic vaporizing device can be used as a smoking cessation device, medicine vaporizing device, etc. Its basic task is to provide vaporizing process and convert the to-be-vaporized liquid, medicine liquid, or other to-be-vaporized material and the like stored in the electronic vaporizing device into vapor fog, steam or aerosol, vapor, etc.

During operation, in order to trigger the power switch and control power on and off of the vaporizing unit, an existing electronic vaporizing device usually continuously detects or senses the air flow through the air inlet channel by the microphone or the air pressure sensor. In such a case, the vaporizing unit can be activated to produce vapor or aerosol upon user's inhale action.

An air pressure difference threshold for activating existing electronic vaporizing device when it is in a power off or sleep state is the same as that for triggering the power switch when it is in a standby state. Thus, false triggering may occur in some particular situations such as environment air pressure change during transportation, or due to misuse by children, resulting power consumption or other possible damage caused by automatic activation.

Furthermore, instead of automatically entering the power off or sleep state, some existing electronic vaporizing device stays in the standby state for too long after inhaling, causing too much power consumption. Or it may enter the sleep state, but the microphone or air pressure sensor still continuously detects the air flow or air pressure difference at the original working frequency. Thus, such electronic vaporizing device in the sleep state causes too much power consumption as well.

Moreover, some existing electronic vaporizing device uses the microphones or the analog signal air pressure sensors which cannot accurately detect the air pressure difference between the inside and outside of the electronic vaporizing device during use, and cannot automatically adjust the produced vapor or aerosol volume according to the air flow or air pressure difference.

### SUMMARY

### Technical problem

An object of the disclosure is to overcome the above shortcomings and provide an electronic vaporizing device with a digital air pressure sensor chip and a controlling method thereof.

### Technical solutions

The disclosure provides a technical solution as follow. An electronic vaporizing device with a digital air pressure sensor chip comprises a vaporizer part arranged with a vaporizing unit, a power output unit, a microcontroller, and a digital air pressure sensor chip; The power output unit is configured to provide output voltage for the vaporizing unit to operate, the microcontroller is electrically connected with the vaporizing unit, the power output unit, and the digital air pressure sensor chip, respectively; The digital air pressure sensor chip is disposed in the air inlet channel of the vaporizer part or in communication with the air inlet channel; The digital air pressure sensor chip comprises an air pressure sensing unit, an air pressure processing unit, a switching signal output unit, and a digital signal output unit; The air pressure sensing unit is configured to detect the air pressure difference between the air inlet channel and external environment; The air pressure processing unit is configured to arithmetically process the air pressure difference detected by means of the air pressure sensing unit, and transmit the air pressure difference value that is arithmetically processed to the switching signal output unit; The switching signal output unit is configured to output a switch signal to activate the microcontroller which is in a sleep state, when the air pressure difference value reaches the set activation threshold; The digital signal output unit is configured to output the air pressure difference value that is arithmetically processed to the microcontroller; The microcontroller comprises an output switch unit, and the output switch unit is configured to control the power output unit to provide output voltage when the air pressure difference value reaches the set working threshold, and control the power output unit to not provide voltage when the air pressure difference value is lower than the working threshold, wherein the activation threshold is greater than the working threshold.

Preferably, the vaporizing unit may comprise a resistive heating element, or an electromagnetic heating element, or an ultrasonic atomizer plate.

Preferably, the microcontroller may comprise an output adjustment unit, and the output adjustment unit may be configured to control and adjust the magnitude of output power of the power output unit to be scaled in accordance with the magnitude of the air pressure difference value, when the air pressure difference value is greater than the working threshold.

Preferably, the air pressure sensing unit may further comprise a variable frequency cycle detection unit, and the variable frequency cycle detection unit may be configured to operate at a set sleep frequency when the microcontroller is in the sleep state, and operate at a set normal frequency when the microcontroller is in the standby state or working state.

Preferably, the digital air pressure sensor chip may further comprise a first storage unit which stores an activation threshold, a sleep frequency, and a normal frequency, wherein the activation threshold may be set in a range of 200-400 Pa, the sleep frequency may be set in a range of 0.5-5 Hz, and the normal frequency may be set in a range of 10-60 Hz.

Preferably, the microcontroller may further comprise a second storage unit which stores a working threshold, and the working threshold may be set in a range of 50-200 Pa.

Preferably, the microcontroller may comprise a wake-up unit electrically connected with the switching signal output unit, and the wake-up unit may be configured to wake the microcontroller up when receiving a switch signal from the switching signal output unit.

Preferably, the wake-up unit may be electrically connected with the vaporizing unit, and the wake-up unit may be configured to control the microcontroller to enter the sleep state when the vaporizing unit enters the standby state for a period of standby time that reaches a set standby time threshold.

The disclosure provides another technical solution as follow. A controlling method of an electronic vaporizing device with a digital air pressure sensor chip, comprising:

I. Setting an air pressure difference activation threshold inside the digital air pressure sensor chip; setting its working frequency when the electronic vaporizing device is in sleep state as the sleep frequency; setting its working frequency when the electronic vaporizing device is in standby or working state as the normal frequency; setting the working threshold of the air pressure difference and setting the standby time threshold in the microcontroller, wherein the activation threshold is greater than the working threshold;

II. When the electronic vaporizing device is in the sleep state, configuring the microcontroller and the digital air pressure sensor chip in such a manner that, the microcontroller is in the sleep state, and the digital air pressure sensor chip operates at a set sleep frequency to cyclically detect the air pressure difference between the air inlet channel and external environment, and compares the detected air pressure difference value with the set activation threshold; and if the air pressure difference value reaches the set activation threshold, the digital air pressure sensor chip outputs a switch signal to the microcontroller to wake the microcontroller up to work, such that the electronic vaporizing device enters the standby state;

III. When the electronic vaporizing device is in the standby state, configuring the digital air pressure sensor chip and the microcontroller in such a manner that, the digital air pressure sensor chip operates at a set normal frequency to cyclically detect the air pressure difference between the air inlet channel and external environment, and outputs the detected air pressure difference value to the microcontroller, the microcontroller compares the air pressure difference value with the set working threshold; and if the air pressure difference value reaches the set working threshold, the microcontroller controls the power output unit to provide voltage to the vaporizing unit such that the electronic vaporizing device enters the working state; meanwhile, the microcontroller calculates the period of standby time, and once the period of standby time exceeds the set standby time threshold, the microcontroller controls the electronic vaporizing device to enter the sleep state;

IV When the electronic vaporizing device is in the working state, configuring the digital air pressure sensor chip and the microcontroller in such a manner that, the digital air pressure sensor chip operates at a set normal frequency to cyclically detect the air pressure difference between the air inlet channel and external environment, and outputs the detected air pressure difference value to the microcontroller, the microcontroller compares the air pressure difference value with the set working threshold; and when the air pressure difference value is lower than the set working threshold, the microcontroller controls the power output unit to not provide output voltage, such that the electronic vaporizing device stops operation and enters back the standby state.

Preferably, the method further comprises:
When the electronic vaporizing device is in the working state, configuring the microcontroller in such a manner that, the microcontroller outputs a controlling signal to the power output unit in proportion to the magnitude of the air pressure difference value, such that the greater the air pressure difference value, the greater the output power.

Preferably, the method further comprises:
setting the sleep frequency in a range of 0.5-5 Hz, setting the normal frequency in a range of 10-60 Hz, setting the activation threshold in a range of 200-400 Pa, setting the working threshold in a range of 50-200 Pa, and setting the standby time threshold in a range of 3-20 minutes.

### Advantages

The electronic vaporizing device is provided with a digital air pressure sensor chip for detecting air pressure difference. It has both an activation threshold and a working threshold, and the activation threshold is greater than the working threshold so that the users need to apply greater suction to activate the electronic vaporizing device which enters the power off state or the sleep state. In such a case, false triggering which may occur in some particular situations such as environment air pressure change during transportation, or due to misuse by children, may be prevented. Further, power consumption or other possible damage caused by automatic activation of the electronic vaporizing device due to false triggering may be prevented. In addition, the electronic vaporizing device can automatically enter the sleep state to save power consumption. As the digital air pressure sensor chip is configured with a normal frequency and a sleep frequency, and the sleep frequency is set to be much lower than the normal frequency in the sleep state, therefore, the power consumed by the air pressure sensing unit can be greatly reduced when the microcontroller or the electronic vaporizing device is in the sleep state. In such a case, the power consumption can be saved, and service time of the electronic vaporizing device can be increased after charging. In addition, the digital air pressure sensor chip of the electronic vaporizing device according to the disclosure can accurately detect the air pressure difference between the inside and outside of the electronic vaporizing device during working, and can further automatically adjust the produced vapor or aerosol volume according to the air pressure difference by means of the output adjustment unit.

### BRIEF DESCRIPTION OF THE DRAWINGS

FIG. 1 is a Function Block Diagram 1 of an embodiment of an electronic vaporizing device according to the disclosure;
FIG.2 is a Function Block Diagram 2 of an embodiment of an electronic vaporizing device according to the disclosure.

### DETAILED DESCRIPTION OF ILLUSTRATED EMBODIMENTS

The term "electronic vaporizing device", as used herein, refers to a vaporizing device or vaporizing equipment for vaporizing the to-be-vaporized solution or material in liquid or solid state into vapor. It may be used for treatment in the medical field by heating and vaporizing medicine liquid into vapor for patient to vape.

### Embodiment 1

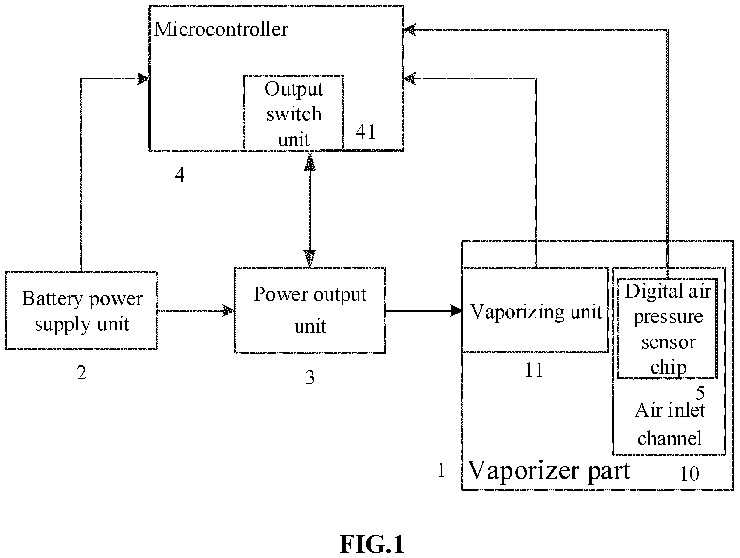

Referring to FIG.1, an electronic vaporizing device with a digital air pressure sensor chip in the embodiment is provided with a vaporizer part 1 arranged with a vaporizing unit 11, a battery power supply unit 2, a power output unit 3, a microcontroller 4, and a digital air pressure sensor chip 5.

Herein, the battery power supply unit 2 is configured to supply power for the power output unit 3, and the power output unit 3 is configured to provide output voltage for the vaporizing unit 11 to operate. When the vaporizing unit 11 is powered on, it vaporizes the to-be-vaporized liquid or other material stored in the electronic vaporizing device. The microcontroller 4 is electrically connected with the vaporizing unit 11, the power output unit 3, and the digital air pressure sensor chip 5, respectively. The digital air pressure sensor chip 5 is disposed in the air inlet channel 10 of the vaporizer part 1, or is connected with the air inlet channel 10. The microcontroller 4 comprises an output switch unit 41, and the output switch unit 41 is electrically connected with the power output unit 3 and is configured to control the power output unit 3 to provide or not provide output voltage.

The digital air pressure sensor chip 5 of the electronic vaporizing device according to the disclosure has two-in-one sensor functions. It is configured to cyclically actively detect the air pressure difference that appears in the air inlet channel 10 when air flow is passing through, and output two signals including a switch signal and an air pressure difference signal depending on the magnitude of the air pressure difference.

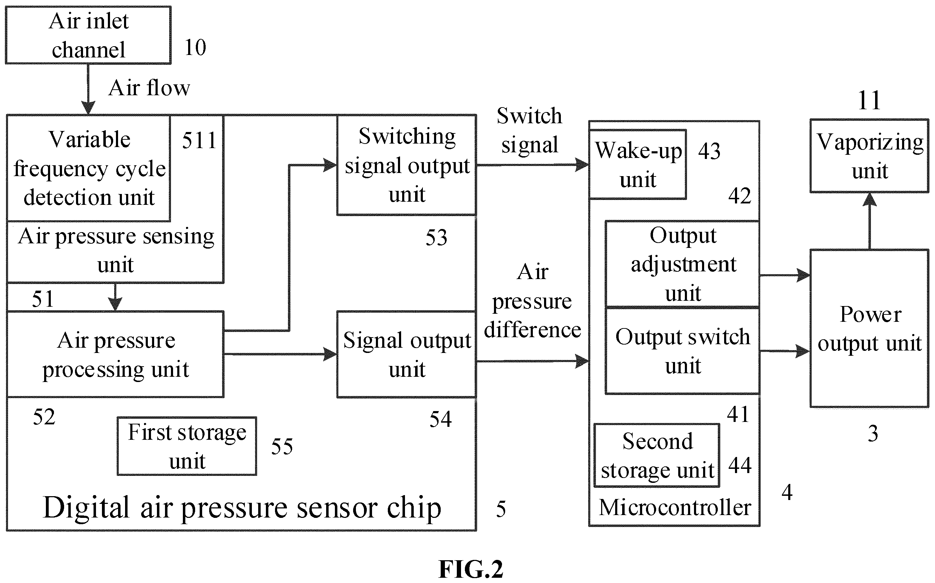

Referring to FIG.2, the digital air pressure sensor chip 5 comprises an air pressure sensing unit 51, an air pressure processing unit 52, a switching signal output unit 53, and a digital signal output unit 54. Herein, the air pressure sensing unit 51 is configured to detect the air pressure difference between the air inlet channel and external environment. The air pressure processing unit 52 is configured to arithmetically process the air pressure difference detected by means of the air pressure sensing unit 51, and transmit the air pressure difference value that is arithmetically processed to the switching signal output unit. The switching signal output unit 53 is configured to output a switch signal for activating the microcontroller 4 which is in a sleep state, when the air pressure difference value reaches the set activation threshold.

When the electronic vaporizing device is in the power off state or the sleep state, the microcontroller 4 does not work. In such a case, only the digital air pressure sensor chip 5 does work, which cyclically actively detects the air pressure difference that appears in the air inlet channel 10 when air flow is passing through. In order to activate the electronic vaporizing device, a user only needs to apply greater suction to vape to make air pressure difference in the air inlet channel of the electronic vaporizing device, such that the microcontroller 4 which is in the sleep state can be activated once the air pressure difference reaches the set activation threshold.

Referring to FIG.2, the signal output unit 54 is configured to output the air pressure difference value that is arithmetically processed to the microcontroller 4. The microcontroller 4 is provided with an output switch unit 41, and the output switch unit 41 is configured to control the power output unit 3 to provide or not provide output voltage for the vaporizing unit 11 when the air pressure difference value reaches or is lower than the set working threshold.

Referring to FIG.2, the digital air pressure sensor chip in the embodiment further comprises the first storage unit 55 which stores an activation threshold, a sleep frequency, and a normal frequency. According to the disclosure, the activation threshold is preferably set in a range of 200-400 Pa, the sleep frequency is preferably set in a range of 0.5-5 Hz, the normal frequency is preferably set in a range of 10-60 Hz.

Referring to FIG.2, the microcontroller 4 further comprises the second storage unit 44 which stores a working threshold, and the working threshold is preferably set in a range of 50-200 Pa.

According to the disclosure, as the activation threshold is higher than the working threshold, it needs more air pressure difference to activate the electronic vaporizing device that is in the sleep state. That is, in order to activate the electronic vaporizing device, the users need to apply greater suction on the electronic vaporizing device. In such a case, false triggering, which may occur in some particular situations such as environment air pressure change during transportation or due to misuse by children, may be prevented. Thus, power consumption or other possible damage of the electronic vaporizing device which may be caused by automatic activation upon false triggering, may be prevented.

Referring to FIG.2, the microcontroller 4 further comprises the output adjustment unit 42, and the output adjustment unit 42 is configured to control and adjust the magnitude of output power of the power output unit 3 to be scaled in accordance with the magnitude of the air pressure difference value when the air pressure difference value is higher than the set working threshold. In such a case, the vapor or aerosol volume produced by the vaporizing unit 11 can be adjusted. That is, the greater the air pressure difference value, the greater the output power and the more vapor or aerosol volume.

The digital air pressure sensor chip of the electronic vaporizing device according to the disclosure can accurately detect the air pressure difference between the inside and outside of the electronic vaporizing device during working, and can further automatically adjust the produced vapor or aerosol volume according to the air pressure difference by means of the output adjustment unit.

The vaporizing unit 11 comprises a resistive heating element, or an electromagnetic heating element, or an ultrasonic atomizer plate. Both the resistive heating element and the electromagnetic heating element may heat the to-be-vaporized liquid or material for vaporizing, while the ultrasonic atomizer plate may vaporize the to-be-vaporized liquid or material by ultrasonic vibration.

Referring to FIG.2, the air pressure sensing unit 51 further comprises a variable frequency cycle detection unit 511, and the variable frequency cycle detection unit 511 is configured to operate at a set sleep frequency (e.g., 1Hz) when the microcontroller 4 is in the sleep state, and operate at a set normal frequency (e.g., 20Hz) when the microcontroller 4 is in the standby state or working state.

The digital air pressure sensor chip is configured with a set sleep frequency and a set normal frequency, and the set sleep frequency may be far lower than the set normal frequency. In such a case, when the microcontroller 4 or the electronic vaporizing device is in the sleep state, the power consumption of the air pressure sensing unit can be greatly reduced, whereby power consumption can be saved and the service time of electronic vaporizing device can be increased after charging. According to the disclosure, the set sleep frequency is preferably in a range of 0.5-5 Hz, the set normal frequency is preferably in a range of 10-60 Hz.

Referring to FIG.2, the microcontroller 4 further comprises a wake-up unit 43 electrically connected with the switching signal output unit 53, and the wake-up unit 43 is configured to wake the microcontroller 4 up when it receives a switch signal from the switching signal output unit 53. The wake-up unit 43 is electrically connected with the vaporizing unit 11, and the wake-up unit 43 is configured to control the microcontroller to enter the sleep state when the vaporizing unit 11 enters the standby state for a period of standby time that reaches a set standby time threshold, e.g., 3 minutes. The period of standby time of the vaporizing unit 11 starts from the moment the vaporizing unit 11 is powered off and stops working. According to the disclosure, the standby time threshold is preferably 3-20 minutes.

### Embodiments of the disclosure

### Embodiment 2

A controlling method of an electronic vaporizing device with a digital air pressure sensor chip in the embodiment comprises steps as follows.

I. Setting an air pressure difference activation threshold inside the digital air pressure sensor chip; setting its working frequency when the electronic vaporizing device is in sleep state as the sleep frequency; setting its working frequency when the electronic vaporizing device is in standby or working state as the normal frequency; setting the working threshold of the air pressure difference and setting the standby time threshold in the microcontroller, wherein the activation threshold is greater than the working threshold;

II. The microcontroller and the digital air pressure sensor chip are configured in such a manner that, when the electronic vaporizing device is in the sleep state, the microcontroller is in the sleep state, and the digital air pressure sensor chip operates at a set sleep frequency to cyclically detect the air pressure difference between the air inlet channel and external environment, and compares the detected air pressure difference value with the set activation threshold; and when the air pressure difference value reaches the set activation threshold, the digital air pressure sensor chip outputs a switch signal to the microcontroller to wake the microcontroller up to work, such that the electronic vaporizing device enters the standby state.

III. The digital air pressure sensor chip and the microcontroller are configured in such a manner that, when the electronic vaporizing device is in the standby state, the digital air pressure sensor chip operates at a set normal frequency to cyclically detect the air pressure difference between the air inlet channel and external environment, and outputs the detected air pressure difference value to the microcontroller, the microcontroller compares the air pressure difference value with the set working threshold; and when the air pressure difference value reaches the set working threshold, the microcontroller controls the power output unit to provide output voltage for the vaporizing unit such that the vaporizing unit is energized to operate and the electronic vaporizing device enters the working state; meanwhile, the microcontroller calculates the period of standby time, and once the period of standby time exceeds the set standby time threshold, the microcontroller controls the electronic vaporizing device to enter the sleep state.

IV The digital air pressure sensor chip and the microcontroller are configured in such a manner that, when the electronic vaporizing device is in the working state, the digital air pressure sensor chip operates at a set normal frequency to cyclically detect the air pressure difference between the air inlet channel and external environment, and outputs the detected air pressure difference value to the microcontroller, the microcontroller compares the air pressure difference value with the set working threshold; and when the air pressure difference value is lower than the set working threshold, the microcontroller controls the power output unit to not provide output voltage such that the vaporizing unit is powered off and stops operation and the electronic vaporizing device stops operation and enters back the standby state.

In the above steps, the sleep frequency is set in a range of 0.5-5 Hz, the normal frequency is set in a range of 10-60 Hz, the activation threshold is set in a range of 200-400 Pa, the working threshold is set in a range of 100-200 Pa, and the standby time threshold is set in a range of 3-20 minutes.

In addition to the above, a controlling method of the electronic vaporizing device with the digital air pressure sensor chip in a further embodiment further comprises steps as follows.

The microcontroller is further configured in such a manner that, when the electronic vaporizing device is in the working state, the microcontroller outputs a controlling signal to the power output unit in proportion to the magnitude of the air pressure difference value, i.e., the greater the air pressure difference value, the higher the output power.

### Industrial applicability

All the above are merely preferred embodiments of the disclosure. The present invention is intended to cover all modifications and equivalent arrangements those skilled in the art can make according to the technical essence of the present invention.

## Claims

1. An electronic vaporizing device with a digital air pressure sensor chip, **characterized in that**, the electronic vaporizing device comprises a vaporizer part (1) arranged with a vaporizing unit (11), a power output unit (3), a microcontroller (4), and a digital air pressure sensor chip (5), wherein the power output unit (3) is configured to provide output voltage for the vaporizing unit (11) to operate, the microcontroller (4) is electrically connected with the vaporizing unit (11), the power output unit (3), and the digital air pressure sensor chip (5), respectively; and the digital air pressure sensor chip (5) is disposed in an air inlet channel (10) of the vaporizer part (1) or in communication with the air inlet channel (10); wherein the digital air pressure sensor chip (5) comprises an air pressure sensing unit (51), an air pressure processing unit (52), a switching signal output unit (53), and a digital signal output unit (54); the air pressure sensing unit (51) is configured to detect an air pressure difference between the air inlet channel (10) and external environment, the air pressure processing unit (52) is configured to arithmetically process the air pressure difference detected by means of the air pressure sensing unit (51), and transmit an air pressure difference value that is arithmetically processed to the switching signal output unit (53); the switching signal output unit (53) is configured to output a switch signal to activate the microcontroller (4) which is in a sleep state, when the air pressure difference value reaches set activation threshold; and the digital signal output unit (54) is configured to output the air pressure difference value that is arithmetically processed to the microcontroller (4); wherein the microcontroller (4) comprises an output switch unit (41), and the output switch unit (41) is configured to control the power output unit (3) to provide output voltage when the air pressure difference value reaches set working threshold, and control the power output unit (3) to not provide voltage when the air pressure difference value is lower than the working threshold, wherein the activation threshold is greater than the working threshold.

2. The electronic vaporizing device with a digital air pressure sensor chip according to claim 1, wherein the vaporizing unit (11) comprises a resistive heating element, or an electromagnetic heating element, or an ultrasonic atomizer plate.

3. The electronic vaporizing device with a digital air pressure sensor chip according to claim 1, wherein the microcontroller (4) comprises an output adjustment unit (42), and the output adjustment unit (42) is configured to control and adjust a magnitude of output power of the power output unit (3) to be scaled in accordance with a magnitude of the air pressure difference value, when the air pressure difference value is greater than the working threshold.

4. The electronic vaporizing device with a digital air pressure sensor chip according to claim 1, wherein the air pressure sensing unit (51) further comprises a variable frequency cycle detection unit (511), and the variable frequency cycle detection unit (511) is configured to operate at a set sleep frequency when the microcontroller (4) is in the sleep state, and operate at a set normal frequency when the microcontroller (4) is in a standby state or a working state.

5. The electronic vaporizing device with a digital air pressure sensor chip according to claim 4, wherein the digital air pressure sensor chip (5) further comprises a first storage unit (55) which stores the activation threshold, the sleep frequency, and the normal frequency, and the activation threshold is set in a range of 200-400 Pa, the sleep frequency is set in a range of 0.5-5 Hz, and the normal frequency is set in a range of 10-60 Hz.

6. The electronic vaporizing device with a digital air pressure sensor chip according to claim 1, wherein the microcontroller (4) further comprises a second storage unit (44) which stores the working threshold, and the working threshold is set in a range of 50-200 Pa.

7. The electronic vaporizing device with a digital air pressure sensor chip according to claim 1, wherein the microcontroller (4) comprises a wake-up unit (43) electrically connected with the switching signal output unit (53), and the wake-up unit (43) is configured to wake the microcontroller (4) up when the wake-up unit (43) receives a switch signal from the switching signal output unit (53).

8. The electronic vaporizing device with a digital air pressure sensor chip according to claim 7, wherein the wake-up unit (43) is electrically connected with the vaporizing unit (11), and the wake-up unit (43) is configured to control the microcontroller (4) to enter the sleep state if the vaporizing unit (11) enters a standby state for a period of standby time that reaches set standby time threshold.

9. A controlling method of an electronic vaporizing device with a digital air pressure sensor chip, **characterized in that**, the method comprising:
I, setting an activation threshold for an air pressure difference inside a digital air pressure sensor chip (5); setting a working frequency when the electronic vaporizing device is in sleep state as a sleep frequency; setting the working frequency when the electronic vaporizing device is in standby or working state as a normal frequency;
setting a working threshold of the air pressure difference and setting a standby time threshold in a microcontroller (4), wherein the activation threshold is greater than the working threshold;
II, when the electronic vaporizing device is in the sleep state, configuring the microcontroller (4) and the digital air pressure sensor chip (5) in such a manner that,
the microcontroller (4) is in a sleep state, and the digital air pressure sensor chip (5) operates at a set sleep frequency to cyclically detect the air pressure difference between an air inlet channel (10) and external environment, and compares a detected air pressure difference value with the activation threshold that is set; and if the air pressure difference value reaches set activation threshold, the digital air pressure sensor chip (5) outputs a switch signal to the microcontroller (4) to wake the microcontroller (4) up to work, such that the electronic vaporizing device enters the standby state;
III, when the electronic vaporizing device is in the standby state, configuring the digital air pressure sensor chip (5) and the microcontroller (4) in such a manner that, the digital air pressure sensor chip (5) operates at set normal frequency to cyclically detect the air pressure difference between the air inlet channel (10) and external environment, and outputs a detected air pressure difference value to the microcontroller (4), the microcontroller (4) compares the air pressure difference value with set working threshold; and if the air pressure difference value reaches the set working threshold, the microcontroller (4) controls the power output unit (3) to provide voltage to the vaporizing unit (11), such that the electronic vaporizing device enters the working state, meanwhile, the microcontroller (4) calculates a period of standby time, and once the period of standby time exceeds set standby time threshold, the microcontroller (4) controls the electronic vaporizing device to enter the sleep state;
IV, when the electronic vaporizing device is in the working state, configuring the digital air pressure sensor chip (5) and the microcontroller (4) in such a manner that, the digital air pressure sensor chip (5) operates at the set normal frequency, to cyclically detect the air pressure difference between the air inlet channel (10) and external environment, and outputs a detected air pressure difference value to the microcontroller (4), the microcontroller (4) compares the air pressure difference value with the set working threshold; and if the air pressure difference value is lower than the working threshold that is set, the microcontroller (4) controls the power output unit (3) to not provide output voltage, such that the electronic vaporizing device stops operation and enters back the standby state.

10. The controlling method of an electronic vaporizing device with a digital air pressure sensor chip according to claim 9, further comprising:
when the electronic vaporizing device is in the working state, configuring the microcontroller (4) in such a manner that, the microcontroller (4) outputs a controlling signal to the power output unit (3) in proportion to a magnitude of the air pressure difference value, such that the greater the air pressure difference value, the higher the output power.

11. The controlling method of an electronic vaporizing device with a digital air pressure sensor chip according to claim 9, further comprising:
setting the sleep frequency in a range of 0.5-5 Hz, setting the normal frequency in a range of 10-60 Hz, setting the activation threshold in a range of 200-400 Pa, setting the working threshold in a range of 50-200 Pa, and setting the standby time threshold in a range of 3-20 minutes.
